# EUROPEAN PATENT APPLICATION

(11) **EP 4 834 212 A1**
(43) Date of publication of application: **04.11.2026**
(21) Application number: 25173772.2
(22) Date of filing: 30.04.2025
(51) Int. Cl.: A61B 5/024, A61B 5/026, A61B 5/145, A61B 5/1495

(54) **SENSOR DEVICE FOR MEASURING AT LEAST ONE PHYSIOLOGICAL PARAMETER OF A HUMAN AND A METHOD FOR ACTIVATING A SENSOR DEVICE**

(71) Applicant: CARAG AG, 6340 Baar (CH)
(72) Inventor: SCHENK, Daniel, 8910 Affoltern am Albis (CH); NAPOLETANO, Daniel, 8455 Rüdlingen (CH)
(74) Representative: Grünecker Patent- und Rechtsanwälte PartG mbB

(57) **Abstract**

The sensor device (2) of the present invention has a sensing unit (4) with a memory (24). On the memory (24) calibration information is stored that allows an evaluation unit (12) to calibrate the sensors (6, 8, 10) of the sensing unit (4). The present invention ensures therefore that in the sensor device (2) for a specific sensing unit (4) always the correct calibration information is used. Preferably, the memory (24) is embedded in a single connector (22) by which all sensors (6, 8, 10) in the sensing unit (4) are connected correctly to the evaluation unit (12).

## Description

Subject matter of the present invention is a sensor device for measuring at least one physiological parameter of a human and a method for activating such a sensor device.

Various devices for measuring physiological parameters of a human are known. E.g, WO 2016/192958 A1 discloses a system for measuring the flow of blood through the tissue of a human body, particularly through a celebral tissue. From EP 4 454 556 A1 a sensor device is known by which the blood oxygen saturation within tissue of a human is measurable. The reliability of the data provide by the respective measurement is of high importance as wrong measurement results can put humans at great risks. The reliability of the measurement data can be improved by a calibration of the respective sensors. Nevertheless, a reliable calibration of the sensors must be based on the correct calibration information, i.e. the calibration information which is assigned to the sensor(s) used. If the wrong calibration information is used the resulting measurement results are not reliable.

Thus, it is the object of the present invention to provide a sensor device for measuring at least one physiological parameter of a human and a method for activating such a sensor device that ensures the use of correct calibration information.

The above problem is solved by a sensor device for measuring at least one physiological parameter of a human comprising:
a sensing unit comprising a sensor for sensing the at least one physiological parameter;
an evaluation unit adapted to output information on the at least one physiological parameter;
a data connection between the evaluation unit and the sensing unit to transmit at least one signal generated by the sensing unit and relating to the at least one physiological parameter from the sensing unit to the evaluation unit; and
a memory comprising stored calibration information for calibrating the sensor, which calibration information will be taken into consideration by the evaluation unit when outputting the output information;
wherein the sensing unit is provided with the memory.

Depending on the kind of sensor different connections are provided, such as electric conductors, optical fibers and/or mechanical connections (e.g. pressure equalization channels) which are understood as data connections in the context of this document.

The data connection is preferably a connection which is based on a conductor, an optical fiber and/or a pressure channel. These connections are referred to as wired connections in the following. While wireless technology is used in healthcare sensor systems, cabled sensors provide a more stable and reliable data transmission, minimizing interference and signal loss that can occur with wireless devices. They do not require batteries or frequent recharging, ensuring continuous operation without the risk of power failure during critical monitoring. Additionally, wired connections enhance security by reducing the risk of data breaches or unauthorized access, which is a concern with wireless communication in medical environments.

The evaluation unit as used herein can only act as a control unit for the sensor device which provides output information to be analyzed or viewed in a different unit or it can comprise e.g. a display displaying the information on the at least one physiological parameter. The term comprises e.g. the heart frequency, the breathing frequency, the blood pressure, the intracranial pressure, the core temperature, the temperature of a specific organ or body part, such as the brain temperature, the oxygen saturation and/or the glucose level of blood.

The term calibration information as used in this document is to be understood as at least one physical parameter allowing to calibrate the at least one sensor in the sensing unit. Calibration is understood as the comparison of measurement values provided by the respective sensor in comparison to a respective calibration standard of known accuracy. E.g., in case of a thermometer as one of the sensors the calibration information can comprise an offset temperature and/or a gradient of the respective thermometer in comparison to a temperature measuring standard. E.g., in case of a pressure sensor as one of the sensors the calibration information can comprise an offset pressure or the temperature dependency of the respective pressure sensor in comparison to a pressure measurement standard. The respective calibration information is applied to the measurement values to calibrate the output of the at least one sensor.

The sensing unit can be provided in the form of a probe, e.g. a catheter, which allows to position the sensor(s) in the sensing unit at a specific measurement position. Preferably, the sensing unit is part of a catheter to measure physiological parameters of the brain of a human being.

The provision of the sensing unit with the memory that contains the calibration information to calibrate the at least one sensor of the sensing unit improves both the security and the ease of use of the sensor device, as it is not necessary to provide calibration information from a different source. Likewise, the sensing unit is always providing the correct calibration information, such that a miscalibration of the sensors in the sensing unit is avoided. A high standard of security is essential to medical devices that are used e.g. to measure at least one physiological parameter of a human as a malfunction of the sensor device might create a significant health risk for the human being in question.

In particular, the sensing unit is part of a sensing device to measure at least one physiological parameter of the brain of a human. In particular, the calibration information comprises at least one of the following information:
1. the pressure is calibrated in the range of 50-100 mmHG, in relation to the cerebral temperature dependence of the pressure and an offset;
2. the intracarnal temperature is calibrated in the range 30-45 °C; and
3. the cerebral oxygenation (SCO2) is calibrated between 30 and 100 %, the optical output power of the respective sensor is calibrated.

The memory is preferably a read-only memory that prevents the user of the sensor device from altering (perhaps involuntary) the calibration information. The memory is preferably embedded in the sensing unit, i.e. it is preferably not possible to detach the memory from the sensing unit without destroying the memory and/or the sensing unit. In this embodiment the memory is preferably provided as an EEPROM or flash memory which is embedded in the sensing unit, preferably in a connector of the sensing unit which is used to connect the sensing unit to the evaluation unit. The physical connection of the memory with the sensing unit further improves the security of the sensor device as it is not possible to change the memory without damaging the sensing unit or to use wrong calibration information. It is, therefore, impossible to change the calibration information which ensures that the measurements with the sensor device are performed with the correct calibration of the at least one sensor.

Preferably, the memory is accessible through a one wire bus system. The term one wire bus system is understood as a system that provides a wired serial bus e.g. the one designed by Dallas Semiconductor providing low speed data communication and supply voltage over a single conductor. The one wire bus system allows a safe and reliable data transfer from the memory via the memory data connection to the evaluation unit. Furthermore, the respective data connection between the memory and the evaluation unit is easily providable as a single cable connection, and, preferably a ground connection, which allows a reliable data connection which has a low probability of failure. This further enhances the security of the sensor device as the one wire bus system allows a reliable and long lasting connection between the memory and the evaluation unit.

Preferably, the sensing unit comprises a connector by which it is connectable with the evaluation unit, whereas the connector comprises at least the data connection, preferably all necessary connections between the sensing unit and the evaluation unit. The data connection comprises a memory data connection as well which allows to transfer data from the memory to the evaluation unit. This allows to use a single connector for all necessary connections between the evaluation unit and the sensing unit. Instead of having to plug in several connectors, e.g. one for each sensor in the sensing unit or one for each kind of connection necessary to connect to the sensing unit (electrical conductors, optical fibers and/or mechanical connections such as pressure guiding connections) a single connector is used. This enhances both the ease of use for the user and the security as only one connector is to be connected. Misconnections can, thus, be avoided. Preferably the connector is fixedly connected to a probe of the sensing unit, such as a catheter, which comprises the sensor, preferably such that the connection between the probe and the connector cannot be released without destroying the connector and/or the probe. This ensures that the memory provided in the sensing unit is provided with the correct calibration information, even, if the memory is embedded in the connector.

The connection between the connector and the evaluation system is preferably provided as a plug system with male and female connectors. Preferably, the connector and the evaluation unit are designed in such a way that a correct connection between the connector and the evaluation unit is ensured, e.g. by using male and female plugs having a specific geometry that only allows the connection of the plugs in a correct relative orientation to one another.

Preferably, the connector comprises at least one of the following connections: an electrical conductor, a multi-wire conductor, a coaxial conductor, an optical fiber and a mechanical connector. The electrical conductor is preferably a single wire conductor. Electrical conductors, multi-wire conductors and coaxial conductors as well as optical fibers are preferably used as data connections to sensors providing an electrical output, e.g. thermometers, light emitters and light detectors, a microphone, a flow measurement sensor and/or a pressure sensor. A mechanical connection is preferably used e.g. to provide a pressure equalization channel.

Preferably, the memory is provided as a removable storage, in particular as RFID storage or SD card. The provision of the memory and, thus, the calibration information on a removable storage allows to re-calibrate the sensor device and providing the renewed calibration information on the removable storage.

Preferably, the memory comprises a checksum of the calibration information generated using a predetermined checksum algorithm allowing to check the integrity of the calibration data. E.g. in use the checksum is transferred to the evaluation unit as well as the calibration information from the memory. In the evaluation unit a checksum is generated from the calibration information using the predetermined checksum algorithm which is compared to the checksum read from the memory. If these two checksums are identical then the calibration is secure, and the sensor device can be used. If not, the calibration is uncertain and not trustworthy and the sensor device cannot be used or, at least, an error message is to be provided to the user of the sensor device. This further improves the security of the sensor device.

Preferably, the sensing unit comprises at least one of the following sensors: an optical sensor, a pressure sensor, a microphone, a temperature sensor, a flow sensor, a sensor for detecting oxygen metabolism or a sensor for detecting blood flow. The optical sensor comprises preferably a light emitter and a light detector, which emits light of a predetermined characteristic and which detects the light after reflection e.g. from tissue of the human body or after transmitting tissue of the human body. This can be used e.g. to measure the oxygen saturation of blood or the flow of blood through e.g. cerebral tissue. A pressure sensor can be used e.g. to measure the pressure in a certain region of the human body, such as the intracranial pressure. A microphone can be used to detect movement in the body, such as peristalsis sounds or the like. A temperature sensor can be used to detect the temperature of e.g. the brain of the human being. A flow sensor can be used e.g. to measure the blood flow in a certain region or tissue of the human body. A sensor for detecting oxygen metabolism can e.g. measure the oxygen saturation of the blood.

According to a further aspect of the invention, a method for activating a sensor device, in particular a sensor device according to the present invention, is proposed, wherein the sensor device comprises:
- a sensing unit comprising a sensor for sensing the at least one physiological parameter;
- an evaluation unit adapted to output information on the at least one physiological parameter;
- a data connection between the evaluation unit and the sensing unit to transmit at least one signal generated by the sensing unit and relating to the at least one physiological parameter from the sensing unit to the evaluation unit; and
- a memory comprising stored calibration information for calibrating the sensor, which calibration information will be taken into consideration by the evaluation unit when outputting the output information;
wherein the sensing unit is provided with the memory, wherein at start-up the calibration information is provided from the memory to the evaluation unit via a memory data connection which calibrates the sensor based on the calibration information.

The memory data connection is part of the data connection. The term activating the sensor device is to be understood as the activation procedure before the respective measurement of the at least one physiological parameter takes place. The term start-up is to be understood as the phase of activation of the sensor device in which the sensor devices is made ready to perform the measurement of the at least one physiological parameter.

As the memory which contains the calibration information is provided with the sensing unit, and is in particular embedded in the sensing unit, the evaluation unit, though probably usable with different sensing units, is always provided with the calibration information which is relevant for this specific sensing unit. Errors in calibration due to use of the wrong calibration information are, thus, avoided, which is essential when measuring physiological parameters of humans.

Preferably, the evaluation unit is accessing data from the memory using a one wire bus system. A one wire bus system allows a reliable "low tech" connection between the memory and the evaluation unit and ensures, therefore, a secure transfer of the calibration data to the evaluation unit.

Preferably, the memory further comprises a checksum of the calibration information generated using a predetermined checksum algorithm, whereas at start-up the checksum is transferred from the memory to the evaluation unit, whereas in the evaluation unit the checksum algorithm is applied to the calibration information transferred from the memory to generate a calculated checksum, whereas the calculated checksum is compared to the checksum transferred from the memory, whereas a failure mode of the sensor device is initiated in case of differences between the checksum transferred and the calculated checksum. This further increases the security of the sensor device as the calibration information and the data connection between the memory and the evaluation unit can be controlled regarding the correctness of the calibration data transferred. The failure mode can comprise an error message to the user and/or the prohibition of the use of the sensor device or the sensing unit to avoid providing misleading information regarding the at least one physiological parameter.

The details and advantages revealed for the sensor device according to the present invention can be transferred and applied to the method according to the present invention and vice versa. Further features and advantages of the present invention will be described in the following with reference to the figures, which illustrate only examples of embodiments of the present invention. The illustrated and described features may be suitably combined with each other.
- Figure 1:: a schematic view of a sensor device; and
- Figure 2:: a cross section of an example of a connector.

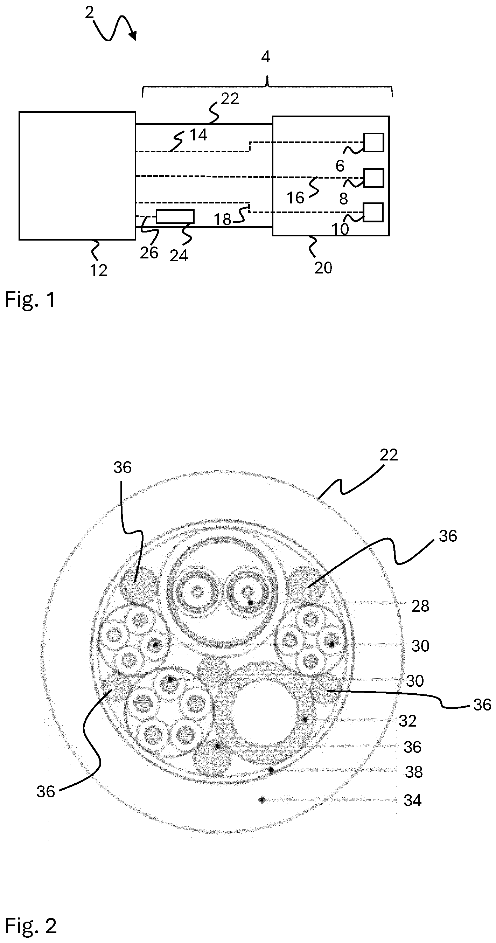

In Fig. 1 an embodiment of a sensor device 2 is shown schematically. The sensor device 2 comprises a sensing unit 4 which comprises a first sensor 6, a second sensor 8 and a third sensor 10. With each of these sensors 6, 8, 10 at least one physiological parameter of a human can be sensed. Examples for such sensors are sensors for measuring intracranial pressure, brain temperature, for measuring parameters of the oxygen metabolism, such as e.g. the tissue oxygen saturation, and/or a cerebral blood flow. In particular the sensing unit 4 can be part of a respective catheter used for monitoring the functionality of a human's brain.

The sensor device 2 comprises furthermore an evaluation unit 12 which is adapted to output information on the at least one physiological parameter based on data provided by the sensors 6, 8, 10. The first sensor 6 is connected to the evaluation unit 12 via a first data connection 14. Depending on the kind of sensor 6, 8, 10 different connections are provided, such as electric conductors, optical fibers and/or mechanical connections (e.g. pressure equalization channels) which are understood as data connections in the context of this document. The second sensor 8 is connected to the evaluation unit 12 via a second data connection 16. The third sensor 10 is connected to the evaluation unit 12 via a third data connection 18. In case of electrical connectors or optical fibers provided as one or more of the data connection 6, 8, 10 the communication between the evaluation unit 12 and the respective sensor 6, 8, 10 is set up as a one wire bus system.

The sensors 6, 8, 10 are preferably mounted in or on a probe 20 which can be in the form of a catheter, e.g. a brain catheter. The sensing unit 4 further comprises a connector 22 which preferably but not mandatory cannot be disconnected from the probe 20 without being damaged.

Alternatively, the probe 20 and the connector 22 are connectable via a plug that is designed such that it ensures a correct connection of the connectors 22 and the respective sensors 6, 8, 10 in the probe 20. In both alternatives misconnection of the sensors 6, 8, 10 and the connector 22 can be avoided.

The sensing unit 4 comprises a memory 24, which is in this embodiment a memory chip embedded into the connector 22. Alternatively, the memory 24 can be embedded in the probe 20. Preferred examples for memories are EEPROMS or flash memories which are embedded in the sensing unit 4, preferably in the connectors 22. The memory 24 is connected via a memory data connection 26 with the evaluation unit 12. The evaluation unit 12 is provided with calibration information for at least one, preferably all, sensors 6, 8, 10 provided in the sensing unit 4. Calibration information is understood as the information necessary to calibrate the at least one sensor 6, 8, 10. Calibration is understood as the comparison of measurement values provided by the respective sensor 6, 8, 10 in comparison to a respective calibration standard of known accuracy.

The data stored in the memory 24 including the calibration information is preferably transferred from the memory 24 to the evaluation unit 12 via a one wire bus system. The term one wire bus system is understood as a system that provides a wired, preferably half-duplex, serial bus designed by Dallas Semiconductor providing low speed data communication and supply voltage over a single conductor. The one wire bus system allows a safe and reliable data transfer from the memory 24 via the memory data connection 26 to the evaluation unit 12. As the memory 24 with the calibration information is implemented to the sensing unit 4 the sensing unit 4 can only be used with the correct calibration information.

Further security measures include the provision of a checksum of the calibration information in the memory 24 which can be transferred to the evaluation unit 12 as well. The checksum is provided using a predetermined checksum algorithm. The evaluation unit 12 can calculate a calculated checksum based on the checksum algorithm from the calibration information transferred from the memory 24 and can compare this calculated checksum with the checksum stored on the memory 24. If both checksums are identical the sensing unit 4 can be used, while in case of a difference between the checksums the evaluation unit 12 initiates a failure mode in which the user of the sensor device 2 is at least informed that a calibration of the sensors 6, 8, 10 cannot be reliably performed. Preferably, in the failure mode does it is not possible to use the sensor device 2 to avoid malfunction.

Fig. 2 shows a cross section of an example of a connector 22. The connector 22 can be understood as a kind of hybrid cable that comprises several data connections such as a coaxial electrical conductor 28, multiwire electrical conductor 30 and/or pressure equalization channels 32. These data connections are encompassed by a cover 34 preferably made from an elastic material. To avoid damaging the conductors 28, 30 and/or the pressure equalization channels 32 the connector 22 comprises one or several fillers 36 which are preferably made from a solid elastic material, preferably having a circular cross section. These fillers 36 are provided alongside or between one or several of the conductors 28, 30 and/or pressure equalization channels 32 to avoid e.g. kinking of the conductors 28, 30 and/or pressure equalization channels 32, thus, avoiding damage of the conductors 28, 30 and/or pressure equalization channels 32. The at least one conductor 28, 30, the at least one pressure equalization channels 32 and the at least one filler 36 are wrapped with a wrapping 38 before applying the cover 34. This minimizes relative motion of the at least one conductor 28, 30, the at least one pressure equalization channels 32 and the at least one filler 36.

The connector 22 allows to connect all sensors 6, 8, 10 needing possibly different kinds of data connections 14, 16, 18 with one connector 22 in the form of a hybrid cable. Preferably, the connector 22 is detachably connectable to the evaluation unit 12 by a connection system (e.g. male/female plugs) that ensures the correct connection of the data connections 14, 16, 18 with respective ports (not shown) of the evaluation unit 12. Therefore, the use of the sensor device 2 has an improved handling compared to devices, in which several connectors are used to connect different sensors 6, 8, 10 of a sensing unit 4 with the evaluation unit 12.

The sensor device 2 of the present invention has a sensing unit 4 with a memory 24. On the memory 24 calibration information is stored that allows an evaluation unit 12 to calibrate the sensors 6, 8, 10 of the sensing unit 4. The present invention ensures therefore that in the sensor device 2 for a specific sensing unit 4 always the correct calibration information is used. Preferably, the memory 24 is embedded in a single connector 22 by which all sensors 6, 8, 10 in the sensing unit 4 are connected correctly to the evaluation unit 12.

### List of reference numerals

- 2: sensor device
- 4: sensing unit
- 6: first sensor
- 8: second sensor
- 10: third sensor
- 12: evaluation unit
- 14: first data connection
- 16: second data connection
- 18: third data connection
- 20: probe
- 22: connector
- 24: memory
- 26: memory data connection
- 28: coaxial electrical conductor
- 30: multiwire electrical
- 32: pressure equalization channel
- 34: cover
- 36: filler
- 38: wrapping

## Claims

1. Sensor device (2) for measuring at least one physiological parameter of a human **comprising:**
- a sensing unit (4) comprising a sensor (6, 8, 10) for sensing the at least one physiological parameter;
- an evaluation unit (12) adapted to output information on the at least one physiological parameter;
- a data connection (14, 16, 18) between the evaluation unit (12) and the sensing unit (4) to transmit at least one signal generated by the sensing unit and relating to the at least one physiological parameter from the sensing unit to the evaluation unit; and
- a memory (24) comprising stored calibration information for calibrating the sensor (6, 8, 10), which calibration information will be taken into consideration by the evaluation unit (12) when outputting the output information;
wherein the sensing unit (4) is provided with the memory (24).

2. Sensor device (2) according to claim 1, **characterized in that** the memory (24) is accessible through a one wire bus system.

3. Sensor device (2) according to one of the preceding claims, **characterized in that** the sensing unit (4) comprises a connector (22) by which it is connectable with the evaluation unit (12), whereas the connector (22) comprises at least the data connection (14, 16, 18).

4. Sensor device (2) according to claim 3, **characterized in that** the connector (22) comprises at least one of the following connections: an electrical conductor (28, 30), a multi-wire conductor (30), a coaxial conductor (28), an pressure equalization channel (32) and a mechanical connector.

5. Sensor device (2) according to one of the preceding claims, **characterized in that** the memory (24) is embedded in the sensing unit (4).

6. Sensor device (2) according to claim 5, **characterized in that** the memory (24) is provided in the form of an EEPROM or a flash storage.

7. Sensor device (2) according to one of claims 1 to 4, **characterized in that** the memory (24) is provided as a removable storage, in particular as RFID storage or SD card.

8. Sensor device (2) according to one of the preceding claims, **characterized in that** the memory (24) comprises a checksum of the calibration information generated using a predetermined checksum algorithm allowing to check the integrity of the calibration data.

9. Sensor device (2) according to one of the preceding claims, **characterized in that** the sensing unit (4) comprises at least one of the following sensors (6, 8, 10): an optical sensor, a pressure sensor, a microphone, a temperature sensor, a flow sensor, a sensor for detecting oxygen metabolism or a sensor for detecting blood flow.

10. Method for activating a sensor device, in particular a sensor device according to one of the preceding claims, wherein the sensor device (2) comprises:
- a sensing unit (4) comprising a sensor for sensing the at least one physiological parameter;
- an evaluation unit (12) adapted to output information on the at least one physiological parameter;
- a data connection (14, 16, 18) between the evaluation unit (12) and the sensing unit (4) to transmit at least one signal generated by the sensing unit (4) and relating to the at least one physiological parameter from the sensing unit (4) to the evaluation unit (12); and
- a memory (24) comprising stored calibration information for calibrating the sensor (6, 8, 10), which calibration information will be taken into consideration by the evaluation unit (12) when outputting the output information;
**characterized in that** the sensing unit (4) is provided with the memory (24), whereas at start-up the calibration information is provided from the memory (24) to the evaluation unit (12) via a memory data connection (26) which calibrates the sensor (6, 8, 10) based on the calibration information.

11. Method according to claim 10, **characterized in that** the evaluation unit (12) is accessing data from the memory (24) using a one wire bus system.

12. Method according to claim 11 or 12, **characterized in that** the memory (24) further comprises a checksum of the calibration information generated using a predetermined checksum algorithm, whereas at start-up the checksum is transferred from the memory (24) to the evaluation unit (12), whereas in the evaluation unit (12) the checksum algorithm is applied to the calibration information transferred from the memory (24) to generate a calculated checksum, whereas the calculated checksum is compared to the checksum transferred from the memory (24), whereas a failure mode of the sensor device (2) is initiated in case of differences between the checksum transferred and the calculated checksum.
